**Europäisches Patentamt**

**European Patent Office**

**Office européen des brevets**

(19)

(11) Veröffentlichungsnummer: **0 212 087 B1**

(12)

# EUROPÄISCHE PATENTSCHRIFT

(45) Veröffentlichungstag der Patentschrift:
**18.10.89**

(21) Anmeldenummer: **86107164.5**

(22) Anmeldetag: **27.05.86**

(51) Int. Cl.⁴: **A 61 F 2/30**, A 61 F 2/34

(54) **Zementfrei im Becken zu verankernde Hüftgelenkspfanne.**

(30) Priorität: **15.07.85 CH 3063/85**

(43) Veröffentlichungstag der Anmeldung:
**04.03.87 Patentblatt 87/10**

(45) Bekanntmachung des Hinweises auf die Patenterteilung:
**18.10.89 Patentblatt 89/42**

(84) Benannte Vertragsstaaten:
**AT BE DE FR GB IT NL SE**

(56) Entgegenhaltungen:
**EP-A- 0 013 863**
**BE-A- 876 941**
**DE-A- 3 205 526**
**DE-A- 3 228 113**
**FR-A- 2 519 545**
**GB-A- 2 080 118**

(73) Patentinhaber: **GEBRÜDER SULZER AKTIENGESELLSCHAFT, Zürcherstrasse 9, CH-8401 Winterthur (CH)**
Patentinhaber: **Protek AG, Stadtbachstrasse 64, CH-3001 Bern (CH)**

(72) Erfinder: **Morscher, Erwin Walter, Prof. Dr.-med., Orthopäd. Uni-Klinik Felix Platter Spital, CH-4055 Basel (CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine zementfrei im Becken zu verankernde Hüftgelenkspfanne aus Kunststoff, insbesondere aus Polyäthylen, aus deren Kontaktfläche zum Knochen, die mit einer das An- und Einwachsen von Gewebe fördernden Struktur versehen ist, mindestens ein zapfenartiger Vorsprung hervorsteht.

Bei zementfreier Verankerung von Kunststoffimplantaten, beispielsweise von Hüftgelenkspfannen aus Polyäthylen, hat man festgestellt, dass der Kunststoff durch Körperflüssigkeiten über lange Zeiträume angegriffen und unter Umständen zersetzt wird. Man ist daher dazu übergegangen, eine Kunststoff-Hüftgelenkspfanne in einen Ring oder eine Schale aus Metall oder Keramik einzubetten, die sich ihrerseits durch An- und/oder Einwachsen von Gewebe mit dem Knochen fest verbindet (DE-C-2 411 617; US-A-3 840 904).

Diese Lösung des geschilderten Problems hat den Nachteil, dass dadurch die Elastizität des Implantates gegenüber dem Knochen verloren geht.

Weiterhin sind eine Vielzahl von Konstruktionen für Hüftgelenkspfannen bekannt, bei denen aus der dem Knochen zugewandten Pfannenoberfläche zapfenartige Vorsprünge vorstehen, die zur Verankerung der Pfanne in entsprechende Bohrungen im Becken eingepresst werden. Diese Vorsprünge sind ein in den Pfannenkörper integrierter Bestandteil der Pfanne, so dass sie bei aus Kunststoff gefertigten Pfannen ebenfalls aus Kunststoff bestehen (siehe z.B. CH-A-644 911; EP-A-0 058 753; DE-C-3 341 724).

Aufgabe der Erfindung ist es, eine Hüftgelenkspfanne zu schaffen, bei der direkte Kontakte von Knochen und Kunststoff möglichst unterbunden sind, ohne dass die Elastizität der Hüftgelenkspfanne aus Kunststoff, die wegen ihrer guten Gleiteigenschaften nach wie vor bevorzugt wird, unzulässig beeinträchtigt wird. Diese Aufgabe wird mit der Erfindung dadurch gelöst, dass die Kontaktfläche der Pfanne mit einer mindestens zweilagigen gitterartigen Metallstruktur bedeckt ist, und dass ferner der Vosprung ebenfalls aus Metall besteht und in den Kunststoff des Pfannenkörpers eingeschweisst ist.

Ein — beispielsweise aus Titan oder einer Titanlegierung bestehendes — Metallgitter, das vorzugsweise als ein Drahtgewebe ausgebildet ist, beeinträchtigt die Elastizität des Implantats nur wenig, da die Stege oder Drähte in ihren Querschnitten jederzeit so gewählt werden können, dass sie das Implantat nicht unzulässig versteifen. Durch die Gitterarmierung werden direkte Kontakte zwischen Kunststoff und Knochen zumindest drastisch vermindert oder sogar praktisch unterbunden. Der oder die zapfenartigen Vorsprünge, die mit Vorteil ebenfalls aus Titan oder einer Titanlegierung bestehen, können am einfachsten durch eine Reibschweissung im Pfannenkörper befestigt werden. Sie sind mit Vorteil als hohlzylindrische Hülsen ausgebildet.

Ein verbessertes Eindringen der Hülsen in den Knochen und eine das Wachstum fördernde Verdichtung der dabei verdrängten Knochensubstanz können erreicht werden, wenn die Hülse an ihrem freien Ende eine trompetenartige Erweiterung ihres Hohlraumes aufweist und in einer scharfen Kante endet.

Für eine Begrenzung der Eindringtiefe des oder der Vorsprünge in den Kunststoff des Pfannenkörpers ist es zweckmässig, wenn der Vorsprung im Fussbereich zu einer Auflageplatte erweitert ist, aus der ein hohler Verankerungszylinder in den Kunststoff des Pfannenkörpers hineinragt; Lockerungen des Vorsprungs in dem Kunststoff kann vorgebeugt werden, wenn die Auflageplatte und/oder der Verankerungszylinder mit mulden- und/oder rillenartigen Vertiefungen versehen sind und/oder wenn der hohle Verankerungszylinder in seinem Mantel mit Durchbrüchen versehen ist. Mulden- oder rillenartige Vertiefungen in der Auflagefläche der Platte und/oder Durchbrüchen in dem Verankerungszylinder erschweren z.B. ein Verdrehen des Vorsprungs sowie Bewegungen infolge von Scherkräften, während die zwischen rillenartigen Vertiefungen verbleibenden Rippen gegebenenfalls zusammen mit den Durchbrüchen im Verankerungszylinder den Widerstand gegen Bewegungen in Achsrichtung des Vorsprungs erhöhen.

Die Befestigung des Gitters auf der Kontaktfläche des Kunststoffes, der vorzugsweise eine der in der Implantat-Technik bekannten und vielfach verwendeten Polyäthylen-Modifikationen ist, erfolgt in einfacher Weise dadurch, dass das Gitter in den durch erhöhte Temperatur erweichten Kunststoff mit Hilfe eines erhöhten Druckes eingepresst wird. Die Temperaturerhöhung ist dabei auf Temperaturen unterhalb der Kristallit-Schmelztemperatur (135 - 140°C) begrenzt; der erforderliche Druck ist abhängig von der Struktur des Gitters und von der gewählten Temperatur. Er kann beispielsweise bis zu 4 kp/mm$^2$ (= 39,24 N/mm$^2$) betragen.

Um das Ein- und Anwachsen von Gewebe zu fördern, ist es vorteilhaft, wenn das Gitter aus mehr als zwei Lagen besteht, wobei die «Porengrösse» der Gitteröffnungen von Lage zu Lage nach aussen zunehmen kann.

Im folgenden wird die Erfindung anhand eines Ausführungsbeispiels im Zusammenhang mit der Zeichnung näher erläutert.

Fig. 1 ist eine hälftig im Schnitt dargestellte Ansicht der neuen Hüftgelenkspfanne;

Fig. 2 gibt gegenüber Fig. 1 vergrössert einen Ausschnitt aus der äusseren Oberfläche der Pfanne mit einem der zapfenartigen Vorsprünge wieder.

Fig. 3 schliesslich ist gegenüber Fig. 2 nochmals vergrössert ein Längsschnitt durch einen in den Kunststoffkörper eingeschweissten zapfenartigen Vorsprung.

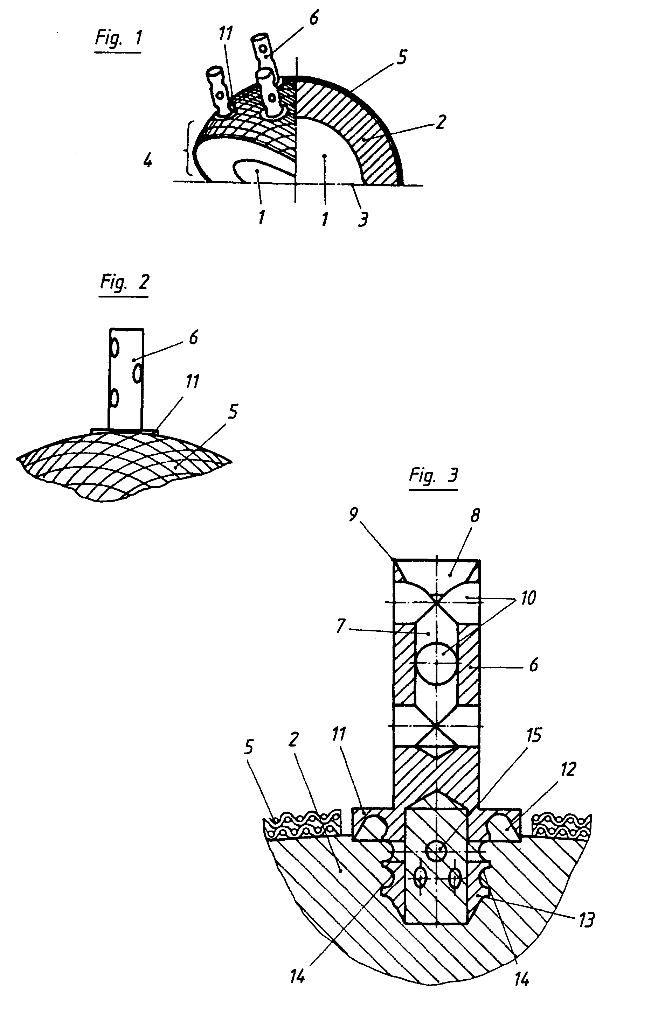

Die den nicht dargestellten Gelenkkopf einer Femurkopfprothese aufnehmende Pfannenschale 1 (Fig. 1) der Hüftgelenkspfanne ist in bekannter Weise in einen im wesentlichen halbkugelförmigen Pfannenkörper 2 aus Kunststoff eingearbeitet; um den Beweglichkeitsbereich der Femurkopfprothese zu erweitern, ist der Pfannenkörper 2 im Bereich des Äquators 3 einseitig schräg abgeschnitten, so dass ihm zur «vollen» Halbkugel ein apfelsinenscheibenartiges Stück 4 fehlt. Erfindungsgemäss ist die Kugeloberfläche des Pfannenkörpers 2, die die Kontaktfläche der Pfanne zum Beckenknochen bildet, von einem mindestens zweilagigen Metallgitter 5 bedeckt, von dem eine Lage zur Verankerung in die Kunststoffoberfläche eingepresst ist.

Im die Abschrägung enthaltenden Sektor ragen aus der Kontaktfläche zum Knochen als zapfenartige Vorsprünge im wesentlichen radial zur Kugeloberfläche drei hohlzylindrische Metallhülsen 6 heraus, die in Form eines Dreiecks angeordnet sind und im wesentlichen in Richtung der Hauptbelastung der Pfanne weisen.

Der Hohlraum 7 (Fig.3) der Hülse 6 endet in einer trompetenartigen Erweiterung 8, wodurch die Hohlzylinderwand der Hülse 6 in einer scharfen Kante 9 ausläuft. Diese dient als «Schneidkante» beim Eintreiben der Hülse 6 in den Beckenknochen, wobei die trompetenartige Erweiterung 8 das «Einfliessen» des dabei verdrängten Knochens in den Holhraum 7 fördert und ein Verdichten dieses Knochengewebes bewirkt. Die Holhzylinderwand der Hülse 6 ist mit Bohrungen 10 versehen, in die Gewebe einwachsen kann.

Pfannenkörperseitig endet die Hülse 6 in einer Auflageplatte 11, die ihre Abstützung auf der Kontaktfläche des Pfannenkörpers 2 gegen in Richtung ihrer Achse wirkende Kräfte vergrössert. Mulden- oder noppenartige Vertiefungen oder Rillen 12 in der Auflagefläche der Platte 11 erhöhen ihre Widerstandsfähigkeit gegen Rotations- bzw. entlang der Oberfläche des Pfannenkörpers wirkende Scherkräfte.

In das Innere des Pfannenkörpers 2 hinein setzt sich die Auflageplatte 11 in einem hohlen Verankerungszylinder 13 fort, der ebenfalls aussen mit Vertiefungen oder Rillen 14 versehen ist und Durchbrüche 15 aufweist.

Die Montage der Hülse 6 im Pfannenkörper 2, die mit dem Gitter 3 nicht verbunden ist, sondern dazu im Bereich der Auflage ein Spiel aufweist, erfolgt durch eine Reibschweissung. Bei dieser erfährt der Kunststoff des Pfannenkörpers 2 eine so starke «Aufweichung», dass er ein Eindringen des Verankerungszylinders 13 ermöglicht und diesen sowie die Bohrungen 15 vollständig ausfüllt.

Der Verankerungszylinder 13 hat vor allem die Aufgabe, die Hülse 6 im Pfannenkörper 2 gegen Kippbelastungen zu fixieren, wobei seine Bohrungen 15 und die Vertiefung 14 die Sicherheit gegen Rotations- und Zugbelastungen erhöhen.

Ebenso wie die Gitter 3 besteht die Hülse 6 aus einem der als besonders gewebefreundlich bekannten Metalle, wie Titan, Tantal, Niob, Zirkon oder Legierungen mit diesen Metallen als Basiswerkstoffen. Sie bilden somit eine gewebefreundliche Oberflächenstruktur, in die im Laufe der Zeit Knochengewebe einwächst, wodurch die Pfanne fest und knochenzementfrei im Knochen verankert wird. Die Maschenweite des Gitters ist dabei mit Vorteil so eng, dass durch sie kein Gewebe hindurch wächst; das Gitter bewirkt daher eine vollständige Abschirmung und Trennung zwischen Knochen und Kunststoff.

**Patentansprüche**

1. Zementfrei im Becken zu verankernde Hüftgelenkspfanne (2) aus Kunststoff, insbesondere aus Polyäthylen, aus deren Kontaktfläche zum Knochen, die mit einer das An- und Einwachsen von Gewebe fördernden Struktur (5) versehen ist, mindestens ein zapfenartiger Vorsprung (6) hervorsteht, dadurch gekennzeichnet, dass die Kontaktfläche der Pfanne mit einer mindestens zweilagigen gitterartigen Metallstruktur (5) bedeckt ist, und dass ferner der Vorsprung (6) ebenfalls aus Metall besteht und in den Kunststoff des Pfannenkörpers (2) eingeschweisst ist.

2. Hüftgelenkspfanne nach Anspruch 1, dadurch gekennzeichnet, dass der Vorsprung (6) aus einer hohlzylindrischen Hülse (6) besteht.

3. Hüftgelenkspfanne nach Anspruch 2, dadurch gekennzeichnet, dass die Hülse (6) an ihrem freien Ende eine trompetenartige Erweiterung (8) ihres Holhraumes aufweist und in einer scharfen Kante (9) endet.

4. Hüftgelenkspfanne nach Anspruch 1, dadurch gekennzeichnet, dass der Vorsprung (6) im Fussbereich zu einer Auflageplatte (11) erweitert ist, aus der ein hohler Verankerungszylinder (13) in den Kunststoff des Pfannenkörpers (2) hineinragt.

5. Hüftgelenkspfanne nach Anspruch 1, dadurch gekennzeichnet, dass die Befestigung des Vorsprungs (6) durch eine Reibschweissung erfolgt ist.

6. Hüftgelenkspfanne nach Anspruch 1, dadurch gekennzeichnet, dass der Vorsprung (6) und das Gitter (5) aus Titan oder einer Titanlegierung bestehen.

7. Hüftgelenkspfanne nach Anspruch 4, dadurch gekennzeichnet, dass die Auflageplatte (11) und/oder der Verankerungszylinder (13) mit rillenartigen Vertiefungen (12, 14) versehen sind.

8. Hüftgelenkspfanne nach Anspruch 4, dadurch gekennzeichnet, dass der hohle Verankerungszylinder (13) in seinem Mantel mit Durchbrüchen (15) versehen ist.

**Claims**

1. A plastics, more particularly polyethylene, acetabulum (2) to be anchored uncemented in the pelvis, at least one pin-like projection (6) extending out of the socket surface which contacts the bone, such surface having a structure (5) promoting the ongrowth and invasion of tissue, characterised in that the contact surface of the socket is covered by an at least two-layer lattice-like metal structure (5) and the projection (6) is also made of metal and is welded into the plastics of the socket (2).

2. An acetabulum according to claim 1, characterised in that the projection (6) is a hollow cylindrical sleeve (6).

3. An acetabulum according to claim 2, characterised in that the sleeve (6) has a flared widening (8) of its hollow interior at its free end and terminates in a sharp edge (9).

4. An acetabulum according to claim 1, characterised in that the projection (6) widens in its base zone to form a foundation plate (11) from which a hollow anchoring cylinder (13) extends into the plastics of the acetabulum (2).

5. An acetabulum according to claim 1, characterised in that the projection (6) is secured by friction welding.

6. An acetabulum according to claim 1, characterised in that the projection (6) and lattice (5) are made of titanium or a titanium alloy.

7. An acetabulum according to claim 4, characterised in that the foundation plate (11) and/or anchoring cylinder (13) are formed with groove-like recesses (12, 14).

8. An acetabulum according to claim 4, characterised in that the hollow anchoring cylinder (13) is formed with perforations (15) in its envelope.


**Revendications**

1. Calotte (2) d'articulation coxofémorale en matière plastique, notamment en polyéthylène, destinée à être ancrée sans ciment dans le bassin, et dans laquelle au moins une protubérance (6) du type tenon fait saillie au-delà de la surface en contact avec l'os qui est pourvue d'une structure (5) favorisant la croissance tissulaire périphérique et interne, caractérisée par le fait que la surface de contact de la calotte est revêtue d'une structure métallique (5) du type entrelacs comprenant au moins deux couches; et par le fait que, en outre, la protubérance (6) consiste également en un métal et est noyée, par soudage, dans la matière plastique du corps de calotte (2).

2. Calotte d'articulation coxofémorale selon la revendication 1, caractérisée par le fait que la protubérance (6) consiste en une douille cylindrique creuse (6).

3. Calotte d'articulation coxofémorale selon la revendication 2, caractérisée par le fait que la cavité de la douille (6) présente, à son extrémité libre, un évasement (8) du type pavillon de trompette, et s'achève par une arête vive (9).

4. Calotte d'articulation coxofémorale selon la revendication 1, caractérisée par le fait que la protubérance (6) est élargie, dans sa région de base, pour former une plaque d'appui (11) à partir de laquelle un cylindre creux d'ancrage (13) pénètre dans la matière plastique du corps de calotte (2).

5. Calotte d'articulation coxofémorale selon la revendication 1, caractérisée par le fait que la fixation de la protubérance (6) résulte d'un soudage par frottement.

6. Calotte d'articulation coxofémorale selon la revendication 1, caractérisée par le fait que la protubérance (6) et l'entrelacs (5) consistent en du titane ou en un alliage de titane.

7. Calotte d'articulation coxofémorale selon la revendication 4, caractérisée par le fait que la plaque d'appui (11) et/ou le cylindre d'ancrage (13) sont munis de renfoncements (12, 14) du type striure.

8. Calotte d'articulation coxofémorale selon la revendication 4, caractérisée par le fait que le cylindre creux d'ancrage (13) est pourvu d'évidements (15) dans son enveloppe.

*Fig. 1*

11  6  5

2

4

1  1  3

*Fig. 2*

6

11

5

*Fig. 3*

9  8

10

7

6

5  2  11  15  12

14  14  13

5